# EUROPEAN PATENT SPECIFICATION

(11) **EP 2 793 766 B1**
(45) Date of publication and mention of the grant of the patent: **06.04.2016**
(21) Application number: 12751585.6
(22) Date of filing: 06.08.2012
(51) Int. Cl.: A61F 5/02

(54) **POSTURE CORRECTOR**
HALTUNGSKORREKTOR
CORRECTEUR DE POSTURE

(30) Priority: 22.12.2011 GB 201122134
(43) Date of publication of application: 29.10.2014
(73) Proprietor: Warren, David Stephen, London, Greater London W1U 6LE (GB)
(72) Inventor: Warren, David Stephen, London, Greater London W1U 6LE (GB)
(74) Representative: Lamb, Martin John Carstairs
(86) International application number: PCT/GB2012/051896
(87) International publication number: WO 2013/093403

(56) References cited:
- US-A- 5 314 404
- US-A- 5 685 831
- US-A1- 2004 073 987

## Description

This invention relates to a posture corrector for the improvement of posture in an individual.

US 2004/0073987 discloses a safety device for use with a sports helmet.

US 5,685,831 discloses a back brace having interlocking frame members jointed at a first pivotal hinge connection where the two vertically disposed members join the other members.

US 5,314,404 discloses a tethered medical restraint device comprising the features as specified in the preamble of claim 1.

In a first aspect of the invention, there is provided a device comprising three lengths of a substantially inextensible material that are worn as a strap or band around the head, around the pelvis, and as an interconnecting strip over the spine which, when applied, aids in correcting posture. Examples of suitable substantially inextensible materials are stretched crepe bandages, or webasto. Preferably, at least one of the lengths of material is flexible. Preferably, at least one of the lengths of material is less than 5cm in width.

To do this aiding and correcting posture, the invention when worn helps to train an individual to align their spine such that there is a decrease in the strain in the muscles of the body from the skull to the pelvis, i.e. the muscles ranging from the skull to cervical, thoracic, and lumbar spine onto the pelvis, and also from the skull and spine to the scapulae and ribcage as well as from the skull to the clavicles, and the tension in the anterior soft tissues from the skull/jaw to the sternum. The effect of this is to improve mechanics of the spine, chest, and abdomen.

Devices that have been previously designed and constructed do not allow for or encourage the natural motion of the spine to be accommodating or improving but do either tether the body to the device in such a way that normal anatomical function is not encouraged to improve or becomes fixed to the rigid positioning of said designs. This means the stresses that should be minimised by such devices are in fact maintained or worse reinforced by detracting from an anatomical optimum.

When the body is normally considered to be at rest; a good example would be lying flat on your back in bed, or floating on your back in a swimming pool, then the curvatures which are naturally present in the body would be allowed to come as close to their optimum as possible for each individual.

Once you stand or sit, gravity pulls on the body and the groups of muscles that are either intrinsic to the spine i.e. insert directly to it and form a muscular corset which is then reinforced by the extrinsic spinal muscles are placed under strain.

Added to the problem above are the bad habits that we develop in the necessity in modern life to operate computer equipment and other machinery, or simply sitting badly. This is further complicated by poor foundation garments for women, i.e. brassieres, and psychological pressures which can lead to bad posture.

The device comprising the three bands/straps which interconnect when worn by an individual encourage the body to align in such a way once properly adjusted as to revert where possible closer to the anatomical optimum.

The bands are first placed around the head and pelvis as shown in the diagram, and then the interconnecting band is worn such that the wearer is standing looking directly into their eyes in a mirror, their body feels as tall as it can be with as little thoracic and lumbar curves as possible by pulling in the muscles of the neck, thorax and abdomen.

With the straps thus aligned round the head and pelvis, and over the spine, any movement the wearer then makes which does not maintain good anatomical alignment will be conveyed to the skull and pelvis which will cause the wearer to alter their action to minimise the strain which will be felt.

If the body is forced by having an inflexible rod clamped to the spine, then this or straps which try to pull the shoulders back will not act evenly on the spine but will act focally imposing more strain not diminishing it.

The devices described above encourage the body to find a path of least resistance to its most comfortable position, and will re-educate to provide better balance and control of an anatomical optimum.

The joints of the spine when the body is optimally aligned anatomically are under minimal strain, and this is also true for the ribs and sternum. When this is in effect there will also be minimal strain on the intercostal muscles and diaphragm, as well as the soft tissues (muscles etc.) attached to the pelvic girdle.

The posture corrector is designed to hold the wearer in such a way that the body is encouraged to hold to its anatomical optimum. Thus once the wearer has the device on if the body is pulled on mechanically to take it out of its anatomical optimum the wearer will feel drag imposed via the straps. At this point the device will encourage the wearer to re-educate the muscles acting on the spine to rebalance their tone front to back, rotationally, and side to side, such that the muscles will strengthen where they have become weak, and relax where they have become too tight.

The effect of this will be to undertake an exercise class specifically to reposition the body into its optimum anatomical position with minimal external strain.

The device comprises a further length of a substantially inextensible material that is worn over the top of the head and connects to the band around the head at two points. This helps the user to locate the vertical and horizontal planes of the head so that they are close as possible to 90 degrees to each other.

The further length of a substantially inextensible material that is worn over the top of the head connects to the band around the head at the same point as the interconnecting strip over the spine, and at the front of the head. By attaching a strip from the point where the vertical band and the band around the head connect to a point midway in the brow the wearer will be actively aware of the position of the head relative to the spine. It will ensure that the plane that is maintained for the positioning of the head will run along the same plane as the spine ideally placing the head in line with the spine such that the eyes are pointing straight ahead and the semi-circular canals of the ear are at 90 degrees to this plane. This positioning anatomically if it can be reached is the ideal and the wearer would continue to adjust the posture corrector until this is achieved.

At least two of the lengths of material may be slidably connected to each other. The slidable connection allows the wearer to adjust the planes of the bands attaching at the head or pelvis to be as close to 90 degrees as possible the advantage of which is to maintain the head relative to the pelvis on the same horizontal plane. The slidable connection may comprise a loop in one length of material in which the other length of material is provided.

At least two of the lengths of material may be fixedly connected to each other. When the vertical band and either of the bands around the head or pelvis are connected fixedly then it is possible to achieve a relative change in the horizontal plane of the head relative to the pelvis or vice versa if the two are not acting together.

At least one of the lengths of material may be adjustable in length. If the vertical band is adjustable in length then, as the wearer has their posture brought into alignment, an incremental change in length by alteration of their posture can be adapted to whilst keeping the body directed to the anatomical optimum.

In a second aspect of the invention, there is a method comprising providing three lengths of a substantially inextensible material as a strap or band around the head, around the pelvis, and as an interconnecting strip over the spine to aid in correcting posture, the method comprising providing a further length of a substantially inextensible material over the top of the head to connect to the band around the head at two points, wherein the further length of a substantially inextensible material over the top of the head connects to the band around the head at the same point as the interconnecting strip over the spine, and at the front of the head.

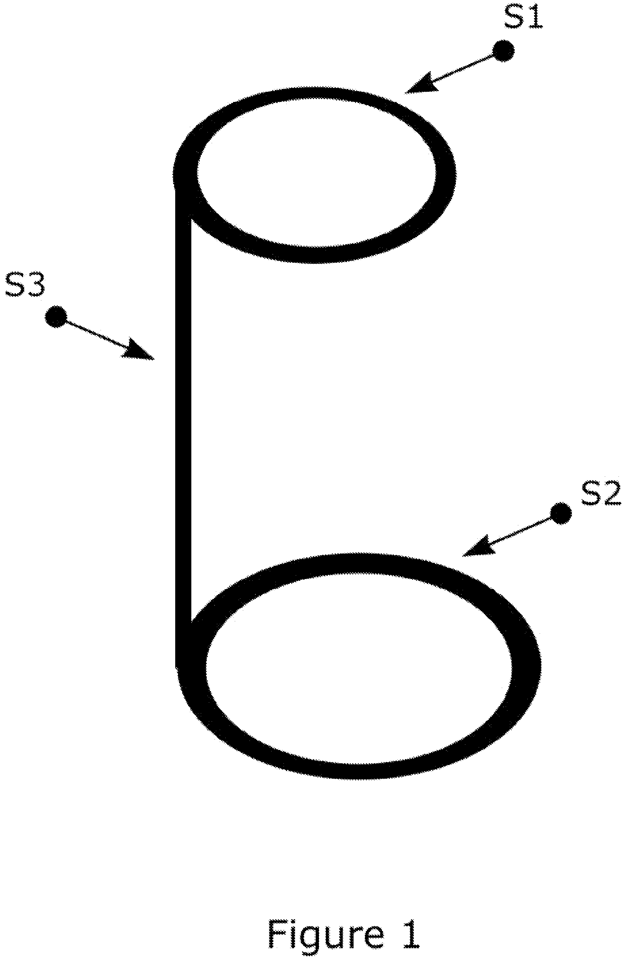

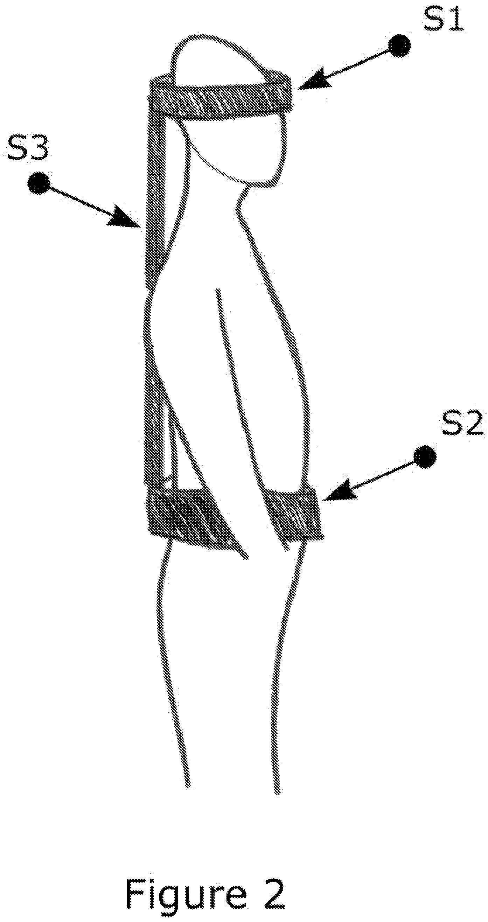

The drawings relate to preferred forms of the invention and are provided by way of example only. Figure 1 shows a posture corrector according to an embodiment of the invention. Figure 2 shows the posture corrector of Figure 1 when applied.

The device as seen in Figure 1 which includes a head strap S1, pelvic strap S2, and connecting strap S3 that runs over the spine attempt to encourage the wearer to regain the spine's anatomical optimum.

These three straps which are adjustable need to be set for each individual so that they are held firmly around the head as per Figure 2, and also for the one around the pelvis, and that the connecting strap is attached to the pelvic strap in alignment over the sacrum and running up over the spine passing under the rear of the head strap, running over the crown of the head to align centrally the spine at the forehead over the user's brow.

The adjustable strap between the skull and the pelvis is then made tight as described above to make the user as tall vertically as they can be, with minimal rotation or lateral movement in the body.

The straps are adjusted in the order head, pelvis and finally spine for the reason the largest sensations will be felt in the head and neck as the positioning of the head on the spine is also affected by the muscles of the jaw and throat. When realigning the spine these structures are only inherently stable between the first and second cervical vertebrae, all the rest of the vertebrae being given stability by a set of stretch and balance receptors within the muscles and ligaments of the spine, as well as in the semi-circular canals of the ears and by one's eyes. For this reason the expression to be as tall vertically in the spine as possible is relevant as at that point the spine will be as close to its optimum for any given individual as possible (with their pre-existing postural problems). Their rotation side bending will mean that to keep the eyes forward and level, these will be brought to a minimum, and with the strap running in line with the spine over the head and down to the middle of the forehead over the users brow the head will be in its optimum position for the wearer to appreciate what muscular change is needed at that present time.

As the body adjusts the individual will be able to feel this and it is this spinal alignment of the connecting strap in communicating to the wearer the body's position.

With the device so adjusted it is then evident to the wearer that they will feel the correct position, that their pelvic girdle needs to be at, a change in their rib mechanics decreasing their breathing effort, and a change in the balance of their lower limbs relative to their spine. These points are normally not felt as the body continually adjusts to decrease strain for one muscle or group relative to its partner, but rarely can an individual appreciate the overall strains that this causes, affecting posture, muscle patternation, that this imposes leading to headaches, jaw atrain, head and shoulder pain, and discomfort. Closer to optimal alignment may also help free the chest and increase drainage of the head and neck i.e. sinus problems.

Once these straps have been adjusted as described, the individual spine will be as near to their optimum as it can be, and the re-educating of their posture and muscle balance will begin and continue. As the body adjusts and the muscle balance improves, the positioning of the straps will need to be adjusted to continue this process.

## Claims

1. A device comprising three lengths (S1, S2, S3) of a substantially inextensible material to be worn as a band around the head, around the pelvis, and as an interconnecting strip over the spine which, when applied, aids in correcting posture, the device comprising a further length of a substantially inextensible material to be worn over the top of the head and connecting to the band to be worn around the head at two points, **characterised in that**
the further length of a substantially inextensible material to be worn over the top of the head connects to the band to be worn around the head at the same point as the interconnecting strip to be worn over the spine, and at the front of the head.

2. A device according to Claim 1, wherein at least two of the lengths (S1, S2, S3) of material are slidably connected to each other.

3. A device according to Claim 1 or 2, wherein at least two of the lengths (S1, S2, S3) of material are fixedly connected to each other.

4. A device according to any preceding Claim, wherein at least one of the lengths (S1, S2, S3) of material is adjustable in length.

5. A method comprising providing three lengths (S1, S2, S3) of a substantially inextensible material as a band around the head, around the pelvis, and as an interconnecting strip over the spine to aid in correcting posture, the method comprising providing a further length of a substantially inextensible material over the top of the head to connect to the band around the head at two points, wherein the further length of a substantially inextensible material over the top of the head connects to the band around the head at the same point as the interconnecting strip over the spine, and at the front of the head.

6. A method according to Claim 5, wherein at least two of the lengths (S1, S2, S3) of material are slidably connected to each other.

7. A method according to Claims 5 or 6, wherein at least two of the lengths (S1, S2, S3) of material are fixedly connected to each other.

8. A method according to any one of Claims 5 to 7, wherein at least one of the lengths (S1, S2, S3) of material is adjustable in length.

## Patentansprüche

1. Vorrichtung, die drei Längen (S1, S2, S3) aus einem im Wesentlichen undehnbaren Material aufweist, das als ein Band um den Kopf, um das Becken und als ein verbindender Streifen über der Wirbelsäule getragen werden soll, die, wenn sie zur Anwendung gebracht wird, die Korrektur der Haltung unterstützt, wobei die Vorrichtung eine weitere Länge eines im Wesentlichen undehnbaren Materials aufweist, die über dem oberen Teil des Kopfes getragen werden soll und eine Verbindung zu dem um den Kopf herum getragenen Band an zwei Stellen aufweist, **dadurch gekennzeichnet, dass**
die weitere Länge eines im Wesentlichen undehnbaren Materials, die über dem oberen Teil des Kopfes getragen werden soll, eine Verbindung zu dem um den Kopf herum getragenen Band an der gleichen Stelle wie der verbindende Streifen herstellt, der über der Wirbelsäule getragen werden soll, und an der Stirn des Kopfes.

2. Vorrichtung nach Anspruch 1, bei der mindestens zwei der Längen (S1, S2, S3) des Materials verschiebbar miteinander verbunden sind.

3. Vorrichtung nach Anspruch 1 oder 2, bei der mindestens zwei der Längen (S1, S2, S3) des Materials fest miteinander verbunden sind.

4. Vorrichtung nach einem der vorhergehenden Ansprüche, bei der mindestens eine der Längen (S1, S2, S3) des Materials in der Länge regulierbar ist.

5. Verfahren, das den folgenden Schritt aufweist: Bereitstellen von drei Längen (S1, S2, S3) aus einem im Wesentlichen undehnbaren Material als ein Band um den Kopf, um das Becken und als ein verbindender Streifen über der Wirbelsäule, um die Korrektur der Haltung zu unterstützen, wobei das Verfahren den Schritt des Bereitstellens einer weiteren Länge eines im Wesentlichen undehnbaren Materials über dem oberen Teil des Kopfes aufweist, um eine Verbindung mit dem Band um den Kopf an zwei Stellen herzustellen, wobei die weitere Länge eines im Wesentlichen undehnbaren Materials über dem oberen Teil des Kopfes eine Verbindung mit dem Band um den Kopf an der gleichen Stelle wie der verbindende Streifen über der Wirbelsäule herstellt, und an der Stirn des Kopfes.

6. Verfahren nach Anspruch 5, bei dem mindestens zwei der Längen (S1, S2, S3) des Materials verschiebbar miteinander verbunden sind.

7. Verfahren nach Anspruch 5 oder 6, bei dem mindestens zwei der Längen (S1, S2, S3) des Materials fest miteinander verbunden sind.

8. Verfahren nach einem der Ansprüche 5 bis 7, bei der mindestens eine der Längen (S1, S2, S3) des Materials in der Länge regulierbar ist.

## Revendications

1. Dispositif, comprenant trois longueurs (S1, S2, S3) d'un matériau essentiellement inextensible, destinées à être portées sous forme d'un bandeau autour de la tête, autour du bassin, et comme une bande d'interconnexion au-dessus de la colonne vertébrale, qui, lors de son application, facilite la correction de la posture, le dispositif comprenant une longueur additionnelle d'un matériau essentiellement inextensible destinée à être portée au-dessus du sommet de la tête et connectée au bandeau destiné à être porté autour de la tête au niveau de deux points, **caractérisé en ce que** :
la longueur additionnelle d'un matériau essentiellement inextensible destinée à être portée au-dessus du sommet de la tête est connectée au bandeau destiné à être porté autour de la tête au niveau du même point que la bande d'interconnexion destinée à être portée au-dessus de la colonne vertébrale, et au niveau de l'avant de la tête.

2. Dispositif selon la revendication 1, dans lequel au moins deux des longueurs (S1, S2, S3) de matériau sont connectées de manière coulissante l'une à l'autre.

3. Dispositif selon les revendications 1 ou 2, dans lequel au moins deux des longueurs (S1, S2, S3) de matériau sont connectées de manière fixe l'une à l'autre.

4. Dispositif selon l'une quelconque des revendications précédentes, dans lequel au moins une des longueurs (S1, S2, S3) de matériau est ajustable en longueur.

5. Procédé, comprenant l'étape de fourniture de trois longueurs (S1, S2, S3) d'un matériau essentiellement inextensible sous forme d'un bandeau porté autour de la tête, autour du bassin, et sous forme d'une bande d'interconnexion au-dessus de la colonne vertébrale pour faciliter le correction de la posture, le procédé comprenant l'étape de fourniture d'une longueur additionnelle d'un matériau essentiellement inextensible au-dessus du sommet de la tête, pour connecter le bandeau porté autour de la tête au niveau de deux points, dans lequel la longueur additionnelle de matériau essentiellement inextensible portée au-dessus du sommet de la tête est connectée au bandeau porté autour de la tête au niveau du même point que la bande d'interconnexion portée au-dessus de la colonne vertébrale, et au niveau de l'avant de la tête.

6. Procédé selon la revendication 5, dans lequel au moins deux des longueurs (S1, S2, S3) de matériau sont connectées de manière coulissante l'une à l'autre.

7. Procédé selon les revendications 5 ou 6, dans lequel au moins deux des longueurs (S1, S2, S3) de matériau sont connectées de manière fixe l'une à l'autre.

8. Procédé selon l'une quelconque des revendications 5 à 7, dans lequel au moins une des longueurs (S1, S2, S3) de matériau est ajustable en longueur.
